# EUROPEAN PATENT APPLICATION

(11) **EP 2 165 604 A1**
(43) Date of publication of application: **24.03.2010**
(21) Application number: 08720644.7
(22) Date of filing: 27.03.2008
(51) Int. Cl.: A01N 25/02, A01N 25/22, A01N 43/80, A01P 1/00

(54) **STABLE MICROBIOCIDAL COMPOSITION**

(30) Priority: 31.05.2007 JP 2007144912
(71) Applicant: Chemicrea Inc., Tokyo 103-0022 (JP)
(72) Inventor: FUNATSU, Ryoji, Tokyo 103-0022 (JP)
(74) Representative: Winkler, Andreas Fritz Ernst
(86) International application number: PCT/JP2008/000765
(87) International publication number: WO 2008/146436

(57) **Abstract**

It is intended to stabilize an aqueous solution of isothiazolone compounds, which contain 5-chloro-2-methyl-4-isothiazolin-3-one and 2-methyl-4-isothiazolin-3-one. A nitrate in a proportion of 3% by weight to 25% by weight and a haloacetamide in a proportion of 5ppm to 500ppm are added to the aqueous solution of the isothiazolone compounds, which contain 5-chloro-2-methyl-4-isothiazolin-3-one and 2-methyl-4-isothiazolin-3-one.

## Description

### [Technical Field]

This invention relates to a microbicidal composition. This invention particularly relates to a novel microbicidal composition, in which stability of 3-isothiazolone compounds in an aqueous solution has been improved, the 3-isothiazolone compounds being useful for antiseptic processing, germicidal processing, and bacteriostatic processing of various aqueous type dispersions in various industrial fields.

### [Background Art]

A wide variety of kinds of germicides and germicidal compositions have heretofore been used in aqueous type dispersion products and aqueous systems in processes for producing the aqueous type dispersion products, such as adhesive agents, resin emulsions, ink, sizing agents, coating compounds, paper, fibers, and construction materials, which are utilized in various industrial fields, in order to prevent the problems from occurring in that various kinds of microorganisms, such as bacteria, yeast, filamentous fungi, and algae, proliferate and markedly lower the quality and the productivity of the products. Particularly, the 3-isothiazolone compounds are capable of efficiently preventing the proliferation, and the like, of a wide variety of microorganisms under various conditions and have therefore been used widely. Among others, mixtures of 5-chloro-2-methyl-4-isothiazolin-3-one and 2-methyl-4-isothiazolin-3-one (e.g., trade name: ZONEN-F, a 9:1 mixture, supplied by Chemicrea Inc., and trade name: KATHON LX SF25, a 3:1 mixture, supplied by Rohm & Haas Co.) have commercially achieved success.

However, in cases where the mixture of 5-chloro-2-methyl-4-isothiazolin-3-one and 2-methyl-4-isothiazolin-3-one is diluted with water and prepared as a formulation, the problems have heretofore been encountered in that these isothiazolone compounds decompose quickly and are not capable of being furnished reliably as an aqueous solution formulation.

There have heretofore been furnished formulations, the stability of which has been enhanced as metal complexes by the addition of metal salts, such as magnesium salts, calcium salts, or sodium salts, in high concentrations. However, the formulations, in which polyvalent metal salts, such as the calcium salts or the magnesium salts, have been added as stabilizing agents in high concentrations, have the problems in that, in cases where the formulation has been added to a specific aqueous type dispersion, such as an emulsion, the formulation destroys the emulsion phase by the action of the metal ions and causes aggregation to occur. Also, in cases where a monovalent metal salt, such as sodium nitrate, is added as the stabilizing agent, long-term stability has not been capable of being improved sufficiently.

Therefore, there have been proposed formulations (e.g., in Patent Literature 1 and Patent Literature 2), in which an oxide, such as a peroxide or a bromic acid, has been added alone or in combination with a nitric acid metal salt as the stabilizing agent, and formulations (e.g., in Patent Literature 3 and Patent Literature 4), in which a small quantity of a copper salt has been added for imparting the stability. However, the blending of the oxide has the problems in that, in cases where the oxide is blended with a different germicide, the oxide decomposes the different germicide by the oxidizing action, or causes coloring to occur in the formulation. Also, the blending of the heavy metal salt, such as copper nitrate, has the risk of adversely affecting the environment.

Also, there has been proposed an industrial germicidal composition (in Patent Literature 5), which consists of 2-methyl-4-isothiazolin-3-one, a haloacetamide in a proportion of at least 1,000ppm, and a solvent. However, the blending of the haloacetamide in a high concentration into the aqueous isothiazolone solution has the risk in that, in the cases of mixing with a different germicide, the different germicide will be caused to decompose, and has the problems in that turbidity and coloring are caused to arise. Further, the formulation, in which only 2-methyl-4-isothiazolin-3-one has been blended as the active constituent, has the problems in that the formulation exhibits a narrower antimicrobial spectrum and a lower antimicrobial activity than a mixed formulation of 5-chloro-2-methyl-4-isothiazolin-3-one and 2-methyl-4-isothiazolin-3-one.
- [Patent Literature 1]: Japanese Unexamined Patent Publication No. 5 (1993)-170608
- [Patent Literature 2]: Japanese Unexamined Patent Publication No. 11 (1999)-158013
- [Patent Literature 3]: Japanese Unexamined Patent Publication No. 8 (1996)-165284
- [Patent Literature 4]: Japanese Unexamined Patent Publication No. 2000-72608
- [Patent Literature 5]: Japanese Unexamined Patent Publication No. 2005-187347

### [Disclosure of Invention]

### [Problems Which the Invention Aims at Solving]

In view of the above circumstances, the object of the present invention is to provide a novel method capable of stabilizing an aqueous solution of isothiazolone compounds, which contain 5-chloro-2-methyl-4-isothiazolin-3-one and 2-methyl-4-isothiazolin-3-one, over a long period of time without necessity of blending of a metal salt in a high concentration. Another object of the present invention is to provide a novel microbicidal composition, which is stable over a long period of time and has good characteristics.

### [Means for Solving the Problems]

The inventors conducted extensive research for providing a stabilized aqueous solution of isothiazolone compounds, which contain 5-chloro-2-methyl-4-isothiazolin-3-one and 2-methyl-4-isothiazolin-3-one. As a result, the inventors have found that, in cases where a nitrate and a trace quantity of a haloacetamide compound are blended together with an aqueous solution containing a mixture of the aforesaid isothiazolone compounds, each of the isothiazolone compounds is capable of being stabilized significantly in water without a metal salt being blended in a high concentration. The present invention has been made on the basis of the findings described above.

The present invention provides a microbicidal composition, containing:
i) isothiazolone compounds, which contain 5-chloro-2-methyl-4-isothiazolin-3-one and 2-methyl-4-isothiazolin-3-one,
ii) a nitrate in a content falling within the range of 3% by weight to 25% by weight with respect to a total weight of the composition,
iii) a haloacetamide in a content falling within the range of 5ppm to 500ppm with respect to the total weight of the composition, and
iv) water.

The present invention also provides a method of stabilizing an aqueous solution of isothiazolone compounds, which contain 5-chloro-2-methyl-4-isothiazolin-3-one and 2-methyl-4-isothiazolin-3-one, the method comprising adding a nitrate and a haloacetamide to the aqueous solution,
the nitrate being added in a proportion falling within the range of 3% by weight to 25% by weight,
the haloacetamide being added in a proportion falling within the range of 5ppm to 500ppm.

From the view point of high availability, and the like, 2-iodoacetamide should preferably be employed as the haloacetamide. Also, the content of the haloacetamide should preferably fall within the range of 5ppm to 200ppm with respect to the total weight of the composition.

The nitrate should preferably be selected from the group consisting of sodium nitrate, potassium nitrate, lithium nitrate, strontium nitrate, ammonium nitrate, magnesium nitrate, and calcium nitrate. The nitrate should more preferably be a nitric acid monovalent metal salt selected from the group consisting of sodium nitrate, potassium nitrate, and lithium nitrate. Also, the content of the nitrate should more preferably fall within the range of 3% by weight to 15% by weight with respect to the total weight of the composition.

In the present invention, the total content of the isothiazolone compounds should preferably fall within the range of 0.5% by mass to 40% by mass.

The composition in accordance with the present invention is stabler in cases where the composition has a pH value lower than 5. The composition in accordance with the present invention should more preferably have a pH value equal to at most 3.5.

The term "aqueous solution of isothiazolone compounds" as used herein means the solution of the isothiazoline compounds having been dissolved in a solvent, which consists essentially of water. The solvent should preferably contain water in a proportion of at least 90%, should more preferably contain water in a proportion of at least 95%, and should most preferably consist of water alone.

Also, the term "stabilizing an aqueous solution of isothiazolone compounds" as used herein means that the isothiazolone compounds contained in water are stabilized against chemical decomposition.

### [Effects of the Invention]

With the present invention, wherein the nitrate compound and the trace quantity of the haloacetamide compound are blended together with the aqueous solution of the isothiazolone compounds, which contain 5-chloro-2-methyl-4-isothiazolin-3-one and 2-methyl-4-isothiazolin-3-one, the stability of each of 5-chloro-2-methyl-4-isothiazolin-3-one and 2-methyl-4-isothiazolin-3-one in water is capable of being enhanced markedly with the blending of the nitrate in a reduced proportion. Also, since the blending proportion of the haloacetamide is very low, it is possible to avoid the problems with regard to the decomposition of a different germicide by the haloacetamide and the problems with regard to the coloring by the haloacetamide. Therefore, the present invention is capable of providing the novel microbicidal composition, which is capable of being kept in a stable state over a long period of time and which has good activity and good characteristics.

### [Best Mode of Carrying Out the Invention]

The composition in accordance with the present invention contains the mixture of 5-chloro-2-methyl-4-isothiazolin-3-one and 2-methyl-4-isothiazolin-3-one. In cases where the composition in accordance with the present invention contains the two kinds of the 3-isothiazolone compounds described above in combination, better germicidal or antiseptic effects are capable of being obtained.

Each of the 3-isothiazolone compounds described above has been widely used as an industrial germicide and is easily available as an article on the market. Also, a mixed formulation on the market, such as ZONEN-F (a 9:1 mixed formulation, supplied by Chemicrea Inc.) or KATHON LX SF25 (a 3:1 mixed formulation, supplied by Rohm & Haas Co.), is capable of being employed in the present invention.

No limitation is imposed upon the blending ratio of 5-chloro-2-methyl-4-isothiazolin-3-one to 2-methyl-4-isothiazolin-3-one in the isothiazolone compounds used in the present invention. However, the blending ratio, expressed in terms of the weight ratio, of 5-chloro-2-methyl-4-isothiazolin-3-one to 2-methyl-4-isothiazolin-3-one should preferably fall within the range between 50:1 and 1:50, should more preferably fall within the range between 10:1 and 1:10, and should most preferably fall within the range between 5:1 and 1:5. In cases where the blending ratio falls within the range described above, particularly large antiseptic effects are capable of being obtained. However, in so far as the objects of the present invention are capable of being accomplished, the blending ratio is not limited to the range described above.

In the composition in accordance with the present invention, the total content of the isothiazolone compounds should preferably fall within the range of 0.5% by weight to 40% by weight with respect to the total weight of the composition, and should more preferably fall within the range of 5% by weight to 25% by weight with respect to the total weight of the composition. In cases where the total content of the isothiazolone compounds falls within the range described above, particularly large antiseptic effects are capable of being obtained. However, in so far as the objects of the present invention are capable of being accomplished, the total content of the isothiazolone compounds is not limited to the range described above.

Also, in the present invention, as the isothiazolone compounds, the mixture consisting essentially of 5-chloro-2-methyl-4-isothiazolin-3-one and 2-methyl-4-isothiazolin-3-one should preferably be employed. However, in so far as the objects of the present invention are capable of being accomplished, besides the two kinds of the 3-isothiazolone compounds described above, the composition may also contain a different isothiazolone compound, such as 2-n-octyl-4-isothiazolin-3-one, 1,2-benzisothiazolin-3-one, 4,5-dichloro-2-n-octyl-4-isothiazolin-3-one, or N-butyl-1,2-benzisothiazolin-3-one.

The microbicidal composition in accordance with the present invention contains the nitrate and the trace quantity of the haloacetamide as the stabilizing agents. In cases where the trace quantity of the haloacetamide is blended in combination with the nitrate, the quantity of the nitrate necessary for the stabilization is capable of being reduced by half. Further, since the blending proportion of the haloacetamide is very low, it is possible to avoid the problems with regard to the decomposition of a different germicide by the haloacetamide and the problems with regard to the coloring by the haloacetamide in the cases of the blending with a different germicide.

In the present invention, as the nitrate, it is possible to employ an arbitrary water-soluble nitrate. Though not limited, examples of preferable nitrates include sodium nitrate, potassium nitrate, lithium nitrate, strontium nitrate, ammonium nitrate, magnesium nitrate, and calcium nitrate. The nitrate should more preferably be a nitric acid monovalent metal salt selected from the group consisting of sodium nitrate, potassium nitrate, and lithium nitrate. In the present invention, one kind of the nitrate may be used alone, or at least two kinds of the nitrates may be used in combination.

Unexpectedly, with the present invention, each of the isothiazolone compounds in the aqueous solution, particularly 5-chloro-2-methyl-4-isothiazolin-3-one, is capable of being stabilized markedly with the blending of the nitrate in a lower proportion. In the present invention, the content of the nitrate falls within the range of 3% by weight to 25% by weight with respect to the total weight of the composition, should more preferably fall within the range of 3% by weight to 15% by weight with respect to the total weight of the composition, and should most preferably fall within the range of 3% by weight to 12% by weight with respect to the total weight of the composition. If the content of the nitrate is lower than 3% by weight, the isothiazolone compounds will not be capable of being stabilized for a long period of time. If the content of the nitrate is higher than 25% by weight, there will be the risk that the problems with regard to aggregation due to metal ions will arise.

Though not limited, examples of haloacetamides, which may be employed in the present invention, include 2-iodoacetamide, 2-bromoacetamide, 2-fluoroacetamide, and 2-chloroacetamide. Particularly, 2-iodoacetamide should preferably be employed. One kind of the haloacetamide may be used alone, or at least two kinds of the haloacetamides may be used in combination.

In the present invention, since the blending proportion of the haloacetamide is very low, it is possible to avoid the problems with regard to the decomposition of a different germicide by the haloacetamide and the problems with regard to the coloring by the haloacetamide. The content of the haloacetamide falls within the range of 5ppm to 500ppm with respect to the total weight of the composition, should preferably fall within the range of 5ppm to 200ppmwith respect to the total weight of the composition, and should more preferably fall within the range of 5ppm to 100ppm with respect to the total weight of the composition. Alternatively, the blending proportion of the haloacetamide may fall within the range of 25ppm to 500ppm, within the range of 25ppm to 200ppm, or within the range of 25ppm to 100ppm. If the blending proportion of the haloacetamide is higher than 500ppm, the coloring will often occur, and the effects with respect to the cost will not be capable of being kept large. Also, if the blending proportion of the haloacetamide is lower than 5ppm, the isothiazolone compounds will not be capable of being stabilized over a long period of time.

The microbicidal composition in accordance with the present invention takes on the form of the aqueous solution. The microbicidal composition in accordance with the present invention is capable of being prepared with processing, wherein the constituents, such as the isothiazoline compounds, are dissolved in the solvent, which consists essentially of water. The solvent should preferably contain water in a proportion of at least 90%, should more preferably contain water in a proportion of at least 95%, should particularly preferably contain water in a proportion of at least 99%, and should most preferably consist of water alone. No limitation is imposed upon the kind of water, and water may be city water, ion-exchanged water, distilled water, industrial water, or the like.

As a solvent other than water, a water-soluble organic solvent may be blended. The proportion of the water-soluble organic solvent in the entire solvent should preferably be lower than 10%, should more preferably be lower than 5%, and should particularly preferably be lower than 1%. Though not limited, examples of the organic solvents include glycols, such as ethylene glycol, diethylene glycol, triethylene glycol, propylene glycol, and hexylene glycol; glycol ethers, such as methyl cellosolve, phenyl cellosolve, diethylene glycol monomethyl ether, and dipropylene glycol monomethyl ether; esters, such as methyl acetate, 2-ethoxyethyl acetate, and propylene carbonate; and amides, such as dimethylformamide.

The composition in accordance with the present invention is capable of being prepared with an arbitrary known technique for dissolving a germicide. By way of example, the composition in accordance with the present invention is capable of being prepared with processing, wherein the mixture of 5-chloro-2-methyl-4-isothiazolin-3-one and 2-methyl-4-isothiazolin-3-one, the haloacetamide, and the nitrate are dissolved in the blending ratio described above in water or in the mixture of water and the water-soluble organic solvent.

In so far as the objects of the present invention are not obstructed, besides the constituents described above, the composition in accordance with the present invention may further contain salts other than the nitrate, which salts are ordinarily contained in similar types of microbicidal compositions, e.g., sodium chloride, potassium chloride, and magnesium chloride.

Also, in so far as the objects of the present invention are not obstructed, when necessary, the composition in accordance with the present invention may further contain an arbitrary stabilizer, an arbitrary surface-active agent, and an arbitrary buffer agent.

The composition in accordance with the present invention has markedly large stabilization effects in cases where the composition has a lower pH value. Though not limited, the composition in accordance with the present invention should preferably have a pH value lower than 5, should more preferably have a pH value equal to at most 3.5, and should most preferably have a pH value falling within the range of 3.0 to 2.0.

Though not limited, the composition in accordance with the present invention is capable of being utilized appropriately for antiseptic processing, germicidal processing, or bacteriostatic processing of paper making process water in paper and pulp industries; cooling water and washing water in various industrial fields; and various industrial products, such as coating colors, cutting oils, latexes, synthetic resin emulsions, starch slurries, calcium carbonate slurries, sediment-water polymers, heavy oil sludge, metal processing oils, textile oils, paints, stainproofing coating compounds, paper coating liquids, and ballast water.

In cases where the composition in accordance with the present invention is used, the adding proportion of the composition may be set appropriately in accordance with the microorganism concentration and the application object. For example, in cases where the composition in accordance with the present invention is used for aqueous type dispersions, which are utilized in various industrial fields, such as adhesive agents, resin emulsions, ink, sizing agents, coating compounds, paper, fibers, and construction materials, the adding proportion of the composition in accordance with the present invention may fall within the range of approximately 5ppm to approximately 1,000ppm (isothiazolone compounds), and should preferably fall within the range of approximately 20ppm to approximately 500ppm.

### [Examples]

The present invention will further be illustrated by the following non-limitative examples. In the examples described below, 2-iodoacetamide was employed as the haloacetamide. Also, the blending proportion was expressed in terms of % by weight with respect to the total weight of the composition.

### (1) Preparation of microbicidal composition

In accordance with each of blending recipes listed in Table 1 below, each of microbicidal compositions was prepared with the processing, wherein 5-chloro-2-methyl-4-isothiazolin-3-one (Cl-MIT), 2-methyl-4-isothiazolin-3-one (H-MIT), and the nitrate (Comparative Examples), or 2-iodoacetamide (IAA) employed even further (Examples) were dissolved in water. In every case, the pH value of the composition was adjusted at 2.5.

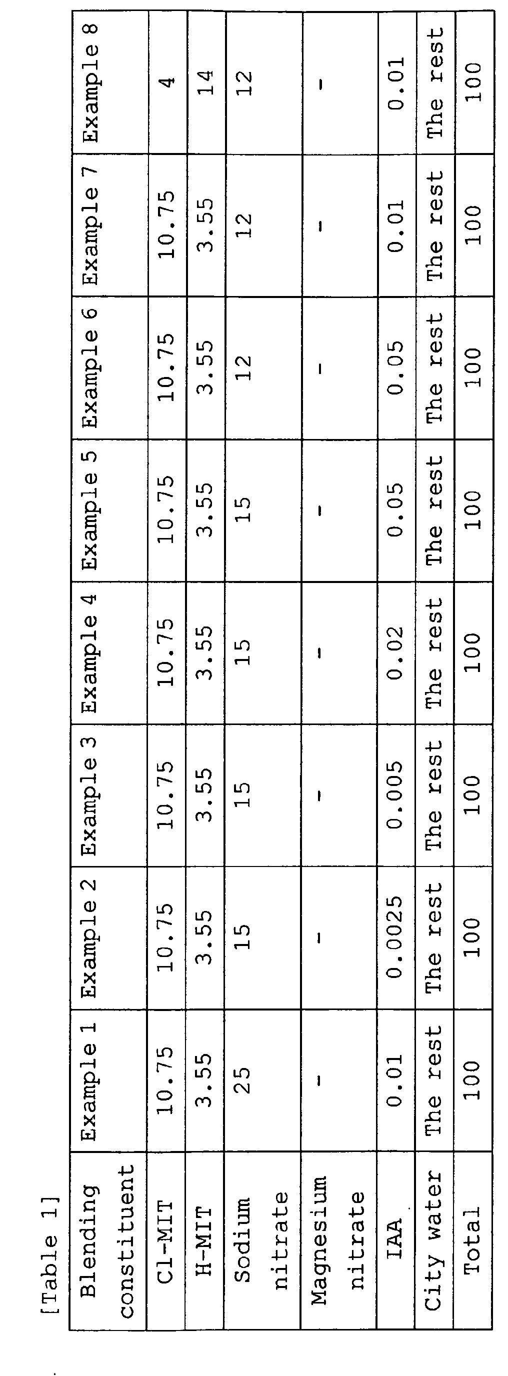

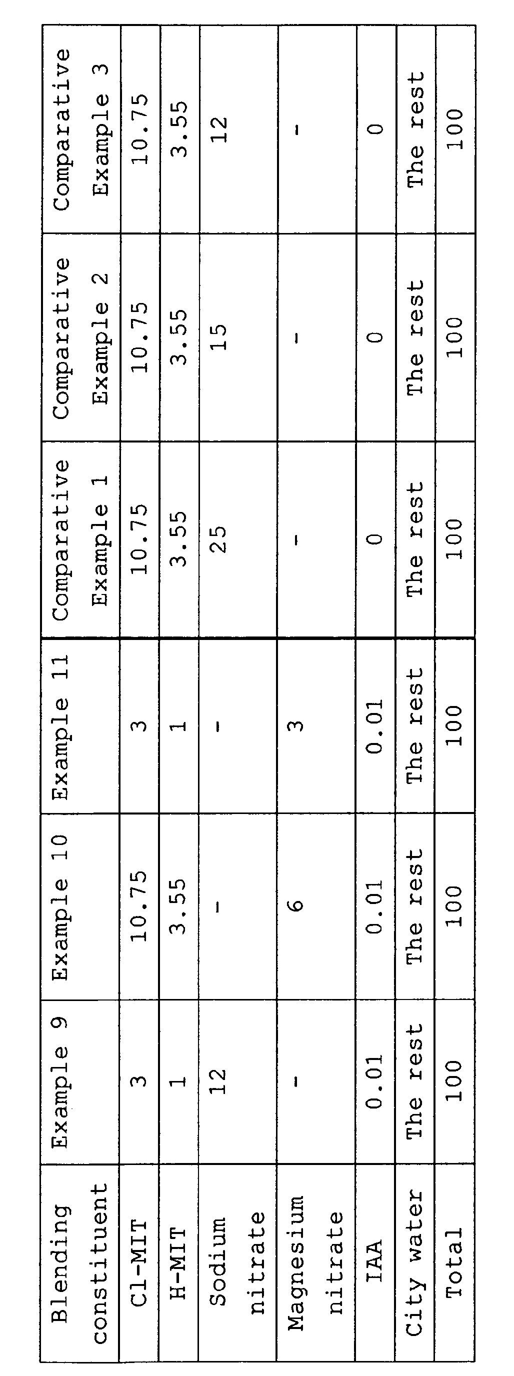

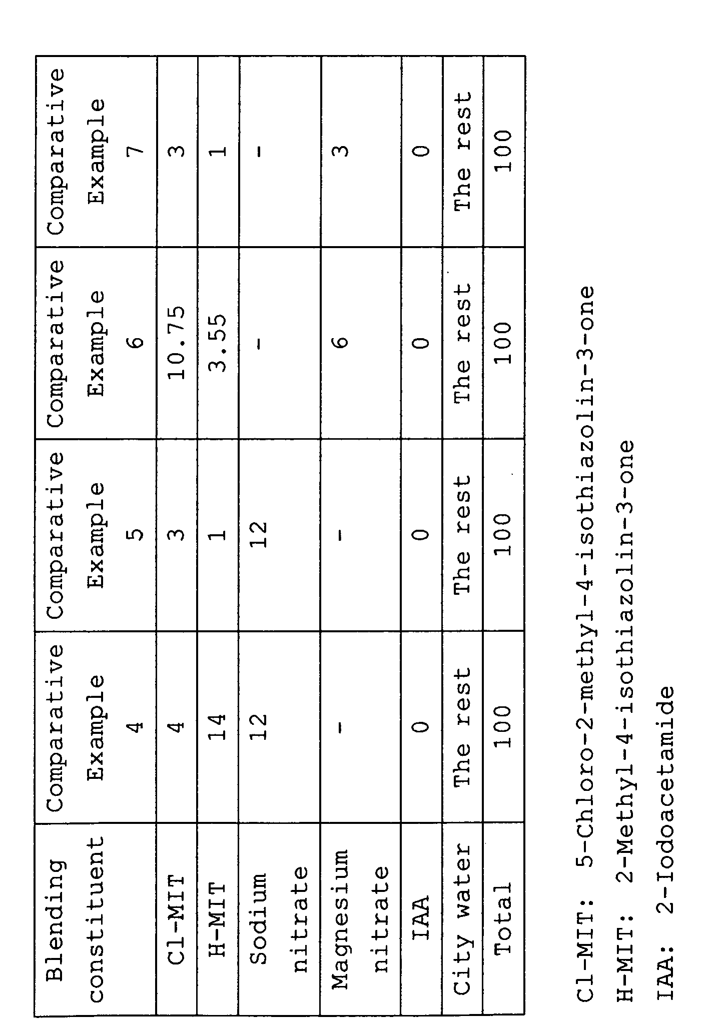

### (2) Storage stability test

With respect to each of the compositions, a storage stability test was made. Each composition was put into a glass vessel and was allowed to stand under the conditions of 50°C. The state was observed with the naked eye with the passage of time. Also, a sample was taken up and subjected to the measurements of the C1-MIT content and the H-MIT content with HPLC (high speed liquid chromatography). Each of residue rates of Cl-MIT and H-MIT was calculated with the measured value, which was obtained at the stage immediately after the test was begun, being taken to be 100.

The results listed in Table 2 were obtained.

**[Table 2]**

| Sample | Cl-MIT residue rate (%) / H-MIT residue rate (%) | | | | Appearance |
|---|---|---|---|---|---|
| | After 7 days | After 14 days | After 21 days | After 30 days | |
| Example 1 | 100/100 | 100/100 | 100/100 | 100/100 | Nearly light yellow transparent solution from just after preparation to test end |
| Example 2 | 100/100 | 100/100 | 100/100 | 100/100 | Nearly light yellow transparent solution from just after preparation to test end |
| Example 3 | 100/100 | 100/100 | 100/100 | 100/100 | Nearly light yellow transparent solution from just after preparation to test end |
| Example 4 | 100/100 | 100/100 | 100/100 | 100/100 | Nearly light yellow transparent solution from just after preparation to test end |
| Example 5 | 100/100 | 100/100 | 100/100 | 100/100 | Nearly light yellow transparent solution from just after preparation to test end |
| Example 6 | 100/100 | 100/100 | 100/100 | 100/100 | Nearly light yellow transparent solution from just after preparation to test end |
| Example 7 | 100/100 | 100/100 | 100/100 | 100/100 | Nearly light yellow transparent solution from just after preparation to test end |
| Example 8 | 100/100 | 100/100 | 100/100 | 100/100 | Nearly light yellow transparent solution from just after preparation to test end |
| Example 9 | 100/100 | 100/100 | 100/100 | 100/100 | Nearly light yellow transparent solution from just after preparation to test end |
| Example 10 | 100/100 | 100/100 | 100/100 | 100/100 | Nearly light yellow transparent solution from just after preparation to test end |
| Example 11 | 100/100 | 100/100 | 100/100 | 100/100 | Nearly light yellow transparent solution from just after preparation to test end |
| Comparative Example 1 | 99/100 | 95/100 | 92/99 | 86/98 | Change from light yellow transparent state to deep yellow transparent state, pressure increase in glass bottle, and gas generation |
| Comparative Example 2 | 98/100 | 90/100 | 88/98 | 80/98 | After one day: generation of turbidity and sediments, pressure increase in glass bottle, and gas generation |
| Comparative Example 3 | 97/100 | 92/100 | 85/100 | 79/99 | After one day: generation of turbidity and sediments, pressure increase in glass bottle, and gas generation |
| Comparative Example 4 | 98/100 | 93/100 | 83/99 | 78/99 | After one day: generation of turbidity and sediments, pressure increase in glass bottle, and gas generation |
| Comparative Example 5 | 96/100 | 94/100 | 84/97 | 80/96 | After one day: generation of turbidity and sediments, pressure increase in glass bottle, and gas generation |
| Comparative Example 6 | 97/100 | 92/100 | 90/98 | 88/98 | After one day: generation of turbidity and sediments, pressure increase in glass bottle, and gas generation |
| Comparative Example 7 | 98/100 | 90/100 | 87/98 | 81/97 | After one day: generation of turbidity and sediments, pressure increase in glass bottle, and gas generation |

| | | | | | |
|---|---|---|---|---|---|
| Cl-MIT: 5-Chloro-2-methyl-4-isothiazolin-3-one H-MIT: 2-Methyl-4-isothiazolin-3-one | | | | | |

As for each of the compositions, in which 3% by weight to 25% by weight of the nitrate and 5ppm to 500ppm of the haloacetamide were blended in combination, each of Cl-MIT and H-MIT was kept in the stable state over 30 days, and an alteration in appearance was not observed.

As for each of the compositions, in which the haloacetamide was not contained or was contained in a proportion lower than 5ppm, and the composition, in which the blending proportion of the nitrate was lower than 3% by weight, Cl-MIT began decomposition after seven days had elapsed, and H-MIT began reduction in quantity after 21 days had elapsed. Also, turbidity and precipitation occurred after one day had elapsed, and gas generation was observed.

Though not illustrated by data, in cases where the pH value of the composition was equal to at least 5.0, there was a tendency of a small quantity of a precipitate to occur with the passage of time. In cases where the pH value took a low value of at most 3.5, markedly large stabilization effects were obtained.

## Claims

1. A microbicidal composition, containing:
i) isothiazolone compounds, which contain 5-chloro-2-methyl-4-isothiazolin-3-one and 2-methyl-4-isothiazolin-3-one,
ii) a nitrate in a content falling within the range of 3% by weight to 25% by weight with respect to a total weight of the composition,
iii) a haloacetamide in a content falling within the range of 5ppm to 500ppm with respect to the total weight of the composition, and
iv) water.

2. A microbicidal composition as defined in Claim 1 wherein the haloacetamide is 2-iodoacetamide.

3. A microbicidal composition as defined in Claim 1 or 2 wherein the content of the haloacetamide falls within the range of 5ppm to 200ppm with respect to the total weight of the composition.

4. A microbicidal composition as defined in Claim 1, 2, or 3 wherein the nitrate is selected from the group consisting of sodium nitrate, potassium nitrate, lithium nitrate, strontium nitrate, ammonium nitrate, magnesium nitrate, and calcium nitrate.

5. A microbicidal composition as defined in Claim 4 wherein the nitrate is selected from the group consisting of sodium nitrate, potassium nitrate, and lithium nitrate.

6. A microbicidal composition as defined in any of Claims 1 to 5 wherein the content of the nitrate falls within the range of 3% by weight to 15% by weight with respect to the total weight of the composition.

7. A microbicidal composition as defined in any of Claims 1 to 6 wherein the total content of the isothiazolone compounds falls within the range of 0.5% by mass to 40% by mass with respect to the total weight of the composition.

8. A microbicidal composition as defined in any of Claims 1 to 7 wherein a pH value of the composition is less than 5.

9. A microbicidal composition as defined in Claim 8 wherein a pH value of the composition is equal to at most 3.5.

10. A method of stabilizing an aqueous solution of isothiazolone compounds, which contain 5-chloro-2-methyl-4-isothiazolin-3-one and 2-methyl-4-isothiazolin-3-one, the method comprising adding a nitrate and a haloacetamide to the aqueous solution,
the nitrate being added in a proportion falling within the range of 3% by weight to 25% by weight with respect to a total weight of the aqueous solution,
the haloacetamide being added in a proportion falling within the range of 5ppm to 500ppm with respect to the total weight of the aqueous solution.

11. A method as defined in Claim 10 wherein the haloacetamide is 2-iodoacetamide.

12. A method as defined in Claim 10 or 11 wherein the adding proportion of the haloacetamide falls within the range of 5ppm to 200ppm with respect to the total weight of the aqueous solution.

13. A method as defined in Claim 10, 11, or 12 wherein the nitrate is selected from the group consisting of sodium nitrate, potassium nitrate, lithium nitrate, strontium nitrate, ammonium nitrate, magnesium nitrate, and calcium nitrate.

14. A method as defined in Claim 13 wherein the nitrate is selected from the group consisting of sodium nitrate, potassium nitrate, and lithium nitrate.

15. A method as defined in any of Claims 10 to 14 wherein the adding proportion of the nitrate falls within the range of 3% by weight to 15% by weight with respect to the total weight of the aqueous solution.

16. A method as defined in any of Claims 10 to 15 wherein the total content of the isothiazolone compounds falls within the range of 0.5% by mass to 40% by mass with respect to the total weight of the aqueous solution.

17. A method as defined in any of Claims 10 to 16 wherein the method comprises adjusting a pH value of the aqueous solution at a value less than 5.

18. A method as defined in Claim 17 wherein the method comprises adjusting a pH value of the aqueous solution at a value equal to at most 3.5.
